# FASCICULE DE BREVET EUROPEEN

(11) **EP 1 021 156 B1**
(45) Date de publication et mention de la délivrance du brevet: **13.02.2002**
(21) Numéro de dépôt: 98943946.8
(22) Date de dépôt: 09.09.1998
(51) Int. Cl.: A61K 7/06, A61K 7/07

(54) **COMPOSITIONS COSMETIQUES DETERGENTES ET UTILISATION**
REINIGENDE KOSMETISCHE ZUSAMMENSETZUNG UND VERWENDUNG
DETERGENT COSMETIC COMPOSITIONS AND USE

(30) Priorité: 25.09.1997 FR 9711958
(43) Date de publication de la demande: 26.07.2000
(73) Titulaire: L'OREAL, 75008 Paris (FR)
(72) Inventeur: DECOSTER, Sandrine, F-93800 Epinay-sur-Seine (FR); BEAUQUAY, Bernard, F-92110 Clichy (FR)
(74) Mandataire: Le Blainvaux Bellegarde, Françoise
(86) Numéro de dépôt international: FR9801922
(87) Numéro de publication internationale: WO9915134

(56) Documents cités:
- EP-A- 0 811 371
- WO-A-94/06403
- WO-A-94/06410
- US-A- 4 529 586
- US-A- 5 667 771

## Description

La présente invention concerne de nouvelles compositions cosmétiques à propriétés améliorées destinées simultanément au nettoyage et au conditionnement des cheveux, et comprenant, dans un support cosmétiquement acceptable, une base lavante constituée de tensioactifs à pouvoir détergent dans laquelle sont également présents à titre d'agents conditionneurs, des polymères cationiques en association avec une silicone aminée particulière. L'invention concerne aussi l'utilisation desdites compositions dans l'application cosmétique susmentionnée.

Pour le nettoyage et/ou le lavage des cheveux, l'utilisation de compositions détergentes (ou shampooings) à base essentiellement d'agents tensioactifs classiques de type notamment anioniques, non-ioniques et/ou amphotères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possédent certes un bon pouvoir lavant, mais les propriétés cosmétiques intrinsèques qui leur sont attachées restent toutefois assez faibles. notamment en raison du fait que le caractère relativement agressif d'un te! traitement de nettoyage peut entraîner à la longue sur la fibre capillaire des dommages plus ou moins marqués liés en particulier à l'élimination progressive ces lipides ou protéines contenues dans ou à la surface de cette dernière,

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes. En outre, sur des cheveux sensibilisés, il est connu d'utiliser de préférence un mélange de silicone et de polymère cationique.

WO94/06403 décrit des compositions de shampooing comprenant un tensioactif anionique, une silicone dispersée insoluble, un polymère cationique et de l'eau. Les silicones aminées citées comprenant des groupements terminaux trialkyl(C1-C6) silyls.

Toutefois, et malgré les progrès réalisés récemment dans le domaine des shampooings à base de polymères cationiques et de silicone, ces demiers ne donnent pas vraiment complètement satisfaction.

En effet, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe) et une insuffisance concemant la brillance.
En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibre affectant le coiffage. Ces inconvénients sont accentués dans le cas de cheveux fins, qui manquent de nervosité et de volume.

Ainsi, un fort besoin existe encore actuellement quant à pouvoir disposer de nouveaux produits présentant, au niveau de l'une ou de plusieurs des propriétés cosmétiques évoqués ci-avant, de meilleures performances.
La présente invention vise à remédier à ces inconvénients.

Ainsi, à la suite d'importantes recherches menées sur la question, il a maintenant été trouvé par la Demanderesse, de façon totalement inattendue et surprenante, qu'en utilisant une silicone aminée particulière et convenablement sélectionnée, telles que définie ci-après, dans des compositions détergentes contenant des polymères cationiques classiques à titre d'agents conditionneurs, il est possible de limiter, voire supprimer, les problèmes généralement liés à l'emploi de telles compositions, à savoir en particulier l'alourdissement, le manque de lissage et de douceur, des cheveux, tout en conservant les autres propriétés cosmétiques avantageuses qui sont attachés aux compositions à base d'agents conditionneur en particulier leur bon pouvoir lavant intrinsèque.

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse.

Toutes ces découvertes sont à la base de la présente invention.

Ainsi, selon la présente invention, il est maintenant proposé de nouvelles compositions détergentes et conditionnantes comprenant, dans un milieu cosmétiquement acceptable, (A) une base lavante et (B) un système conditionneur comprenant au moins un polymère cationique et au moins une silicone aminée de masse moléculaire moyenne en poids comprise entre 11 000 et 25 000 selon la revendication 1.

L'invention a également pour objet l'utilisation en cosmétique des compositions ci-dessus pour le nettoyage et le conditionnement des matières kératiniques et en particulier des cheveux .

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les éléments essentiels rentrant dans la composition des produits selon l'invention sont (A) une base lavante, (B) un système conditionneur comprenant (i) le ou les polymères cationiques, (ii) la ou les silicones aminées particulières.

### A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques; amphotères, non ioniques, zwittérioniques et cationiques.

Toutefois, selon l'invention, la base lavante comprend de préférence des tensioactifs anioniques ou des mélanges de tensioactifs anioniques et de tensioactifs amphotères, et encore plus préférentiellement ne contient que ce type de tensioactif ou mélange de tensioactifs.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 8 % à 35 % en poids, et encore plus préférentiellement de 10 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables. on peut également citer les sels d'acides gras tels que les sels des acides oléique, ricinoléique, palmitique, stearique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 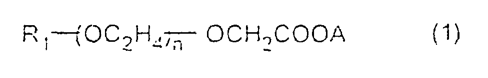 dans laquelle :
R₁ désigne un groupement alkyle ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K. Li, Mg ou un reste monoéthanolamine ou triéthanolamine. On peut également utiliser des mélanges de composés de formule (1) en particulier des mélanges dans lesquels les groupements R₁ sont différents.

Parmi les tensioactifs anioniques, on préfère utiliser selon l'invention les sels d'alkylsulfates et d'alkyléthersufates et leurs mélanges.

### (ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaine grasse comportant par exemple 8 à 18 atomes de carbone, le nombre de groupements oxyde d'éthylène ou oxyde de propylène pouvant aller notamment de 2 à 50 et le nombre àe groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycéroi et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthyléneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaines, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N(R₃)(R₄)(CH₂COO⁻) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C) (3)

dans laquelle :
B représente -CH₂CH₂OX', C représente -(CH₂)_{z} -Y', avec z = 1 ou 2.
X' désigne le groupement -CH₂CH₂-COOH ou un atome d'hydrogène
Y' désigne -COOH ou le radical -CH₂- CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.

### B- SYSTEME CONDITIONNEUR

### (i) Polymère(s) cationique(s) :

Les compositions conformes à l'invention comprennent en outre nécessairement un polymère cationique.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 853.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société Maybroook et dénommnés dans le dictionnaire CTFA "Steartrimonium Hydrolyzed Collagen" ;
- les hydrolysats de protéines animales portant des groupements triméthylbenzylammonium tels que les produits vendus sous la dénomination "CROTEIN BTA" par la Société CRODA et dénommés dans le dictionnaire CTFA "Benzyltrimonium hydrolyzed animal protein" :
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "CROQUAT® L" dont les groupements ammonium quaternaires comportent un groupement alkyle en C₁₂ ;
- le "CROQUAT® M" dont les groupements ammonium quaternairees comportent des groupements alkyle en C₁₀-C₁₈;
- le "CROQUAT® S" dont les groupements ammonium quaternaires comportent un groupement alkyle en C₁₈;
- le "CROTEIN® Q" dont les groupements ammonium quaternaires comportent au moins un groupe alkyle ayant de 1 à 18 atomes de carbone.

Ces différents produits sont vendus par la Société CRODA.

D'autre protéines ou hydrolysats quaternisés sont par exemple ceux répondant à la formule : 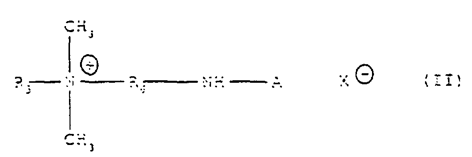 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "LEXEIN® QX 3000", appelé dans le dictionnaire CTFA "Cocotrimonium Collagen Hydrolysate".

On peut encore citer les protéines végétales quaternisées telles que les protéines de blé, de maïs ou de soja : comme protéines de blé quaternisées, on peut citer celles commercialisées par la Société CRODA sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotnticum QS", appelée dans le dictionnaire CTFA "Steardimonium hydrolysed wheat protein".

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylamino-alkyle quaternisés ou non, tels que les produits vendus sous la dénomination "GAFQUAT" par la Société ISP, comme par exemple GAFQUAT® 734, 755 ou HS100 ou bien le produit dénommé "Copolymére 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR® " (JR® 400, JR® 125, JR® 30M) ou "LR® " (LR® 400, LR® 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diallyl diméthyl-ammonium.
   Les produits commercialisés répondant à cette définition sont plus particulièrement les produits vendus sous la dénomination "CELQUAT® L 200" et "CELQUAT® H 100" par la Société NATIONAL STARCH.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR® C13 S, JAGUAR® C 15, JAGUAR® C 17 ou JAGUAR® C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétèrocycliques, ainsi que les produits d'oxydation et/ou de quaternisation de ces polymères. De tels polymères sont notamment décrits dans les brevets français 2.162.025 et 2.280.361;
(6) les polyaminoamides solubles dans l'eau préparés en particulier par polycondensation d'un composé acide avec une polyamine ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE® F, F4 ou F8" par la société SANDOZ.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydnne par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett® 57" par la société Hercules Inc. ou bien sous la dénomination de "PD 170" ou "Delsette® 101" par la société Hercules dans le cas du copolymère d'acide adipique/époxypropyl/diéthylène-triamine.
(9) les cyclopolymères de méthyl diallyl amine ou de diallyl diméthyl ammonium tels que les homopolyméres ou les copolymères comportant comme constituant principal de la chaîne des motifs répondant aux formules (VI) ou (VI') : 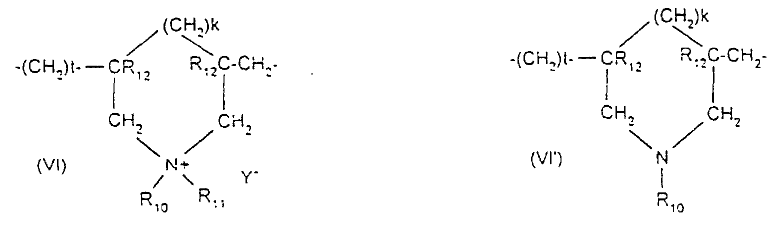 formules dans lesquelles k et t sont égaux à 0 ou 1, la somme k + t étant égale à 1 ; R₁₂ désigne un atome d'hydrogéne ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement les homopolyméres de chlorure de diallyldiméthylammomum de masse moléculaire moyenne en nombre (Mn) comprise entre 5000 et 200000 et plus particulièrement de Mn comprise entre 6000 et 20000.
(10) le polymère de diammonium quaternaire contenant des motifs récurants répondant à la formule : 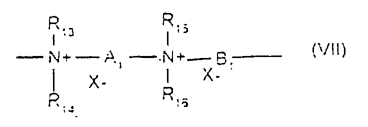 formule (VII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁₋₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH₂)n-CO-D-OC-(CH₂)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)x-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule . -NH-Y-NH-, où Y désigne un radical hydrocarbonè linéaire ou ramifié, ou bien le radical bivalent

      -CH₂-CH₂-S-S-CH₂-CH₂-;
   d) un groupement uréylène de formule : -NH-CO-NH- ;
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 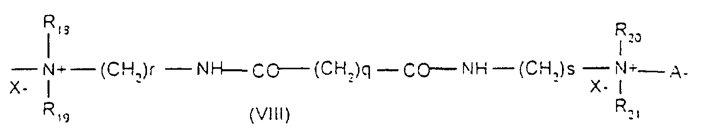 formule dans laquelle :
   R₁₈, R₁₉, R₂₀ et R₂₁, identiques ou différents, représentent un atome d'hydrogène ou un radical méthyle, éthyle, propyle, β-hydroxyethyle, β-hydroxypropyle ou -CH2CH2(OCH2CH2)pOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6.
   q est égal à 0 ou à un nombre entier compris entre 1 et 34,
   X désigne un atome d'halogène,
   A désigne un radical d'un dinalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "MIRAPOL A 15", "MIRAPOL® AD1", "MIRAPOL® AZ1" et "MIRAPOL® 175" vendus par la société MIRANOL.
(12) les homopolyméres ou copolymères dérivés des acides acrylique ou méthacrylique et comportant des motifs : 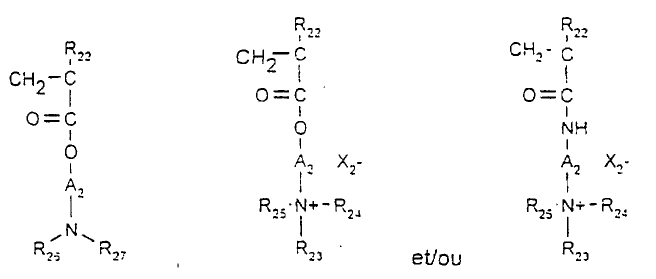 dans lesquels les groupements R₂₂ désignent indépendamment H ou CH₃,
   les groupements A1 désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignant indépendamment un groupe alcoyle de 1 à 18 atomes de carbone ou un radical benzyle,
   les groupements R₂₆ et R₂₇ représentent unatome d'hydrogène ou un groupement alcoyle de 1 à 6 atomes de carbone,
   X₂⁻ désigne un anion, par exemple methosulfate ou halogénure, tel que chlorure ou bromure.
   Le ou les comonomères utilisables dans la préparation des copolyméres correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations LUVIQUAT® FC 906, FC 550 et FC 370 par la société B.A.S.F..
(14) Les polyamines comme le POLYQUART® H vendu par HENKEL, référencé sous le nom de « POLYETHYLENEGLYCOL (15) TALLOW POLYAMINE » dans le dictionnaire CTFA.
(15) Les polymères réticulés de sel (par exemple chlorure) de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide ou le méthacrylate de pentaérythritol. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammomum contenant environ 50 % en poids de l'homopolymère dans de l'huile minérale. Cette dispersion est commercialisée sous le nom de « SALCARE® SC 95 » par la Société ALLIED COLLOIDS.

D'autres polymères cationiques utilisables dans le cadre de l'invention sont des polyalkylèneimines, en particulier des polyéthyièneimines, des polymères contenant des motifs vinylpyridine ou vinylpyridinium, des condensats de polyamines et d'épichlorhydrine, des polyuréyiénes quaternaires et les dérivés de la chitine.

Selon l'invention, on peut utiliser plus particulièrement les polymères choisis parmi le MIRAPOL, le composé de formule (VII) dans laquelle R₁₃, R₁₄, R₁₅ et R₁₆ représentent le radical méthyle, A1 représente le radical de formule -(CH₂)₃- et B1 représente le radical de formule -(CH₂)₆- et X⁻ représente l'anion chlorure et le composé de formule (VII) dans laquelle R₁₃ et R₁₄ représentent le radical éthyle, R₁₅ et R₁₆ représentent le radical méthyle, A1 et B1 représentent le radical de formule -(CH₂)₃- et X⁻ représente l'anion bromure.

Parmi tous les polymères cationiques susceptibles d'être utilisés dans le cadre de la présente invention on préfère mettre en oeuvre les dérivés d'éther de cellulose quaternaires tels que les produits vendus sous la dénomination « JR® 400 » par la société UNION CARBIDE CORPORATION, les cyclopolymères. en particulier les homopolyméres du chlorure de diallydiméthylammonium vendus sous la dénomination « MERQUAT® 100 » et ses homologue de pcids moléculaire inférieur et les copolymères du chlorure de diallyldiméthylammonium et d'acrylamide, vendus sous les dénominations « MERQUAT® 550 » et « MERQUAT S » par la société MERCK, les polysaccharides cationiques et plus particulièrement la gomme de guar modifiée par du chlorure de 2,3-époxypropyl triméthylammonium vendue sous la dénomination « JAGUAR® C13S » par la société MEYHALL.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

### (ii)- Silicones aminées

### (1)- Silicone(s) aminée(s)

Les silicone aminée selon l'invention ont une masse moléculaire moyenne en poids comprise entre 11 000 et 25 000.

Selon l'invention, on désigne par silicone aminée toute silicone comportant au moins une amine primaire, secondaire, tertiaire ou un groupement ammonium quaternaire.

On peut ainsi citer :
(a) les polysiloxanes dénommés dans le dictionnaire CTFA (4^{ème} Ed. 1991) "amodiméthicone" et répondant à la formule : 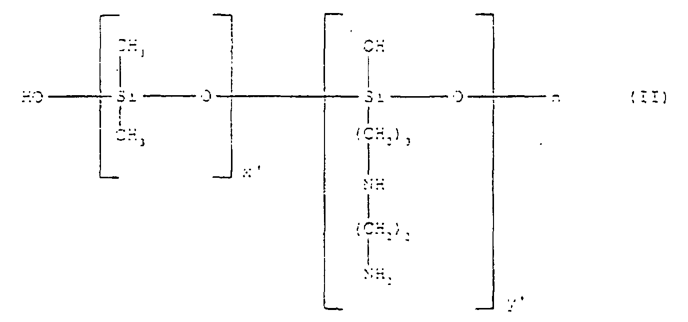 dans laquelle x' et y' sont des nombres entiers dépendant de la masse moléculaire moyenne en poids, telles que ladite masse moléculaire est comprise entre 11 000 et 25 000.
(b) les polysiloxanes dénommés dans le dictionnaire CTFA (7^{ème} Ed. 1997) "amodiméthicone" et répondant à la formule : 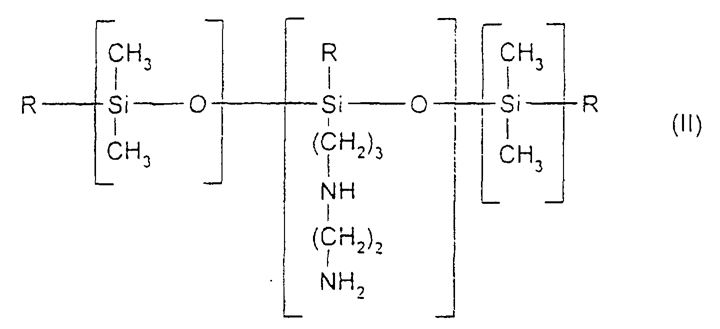 dans laquelle R désigne OH,
   la masse moléculaire moyenne en poids étant comprise entre 11 000 et 25 000.

Lorsque ces silicones aminées sont mises en oeuvre, une forme de réalisation particulièrement intéressante est leur utilisation sous forme d'émulsion huile dans eau. Les tensioactifs peuvent être de toute nature mais de préférence cationiques et/ou non ioniques. On peut utiliser par exemple le produit vendu sous la dénomination "Silsoft TP515 Silicone Emulsion " par la Société OSI qui comprend, outre l'amodiméthicone, un agent de surface cationique comprenant un mélange de produits répondant à la formule : 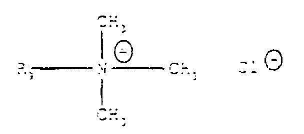 dans lequel R₉ désigne des radicaux alcényle et/ou alkyle ayant de 12 à 20 atomes de carbone et de préférence 16 atomes de carbone,
en association avec un agent de surface non ionique connu sous la dénomination "C₁₁-C₁₅ Pareth-15" qui est mélange d'alcools gras en C₁₁-C₁₅ polyoxyéthylénéné en moyenne par 15 moles d'oxyde d'éthylène.

La taille moyenne des particules de silicone dans l'émulsion est généralement comprise entre 30 nm et 2 microns, de préférence entre 0,1 et 0,5 microns et plus particulièrement entre 0,1 et 0,3 microns.

Les masses moléculaires moyennes en poids de ces silicones aminées sont mesurées par Chromatographie par Perméation de Gel (GPC) à température ambiante en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.

Les compositions conformes à l'invention renferment les silicones aminées définies ci-dessus à des teneurs pondérales qui peuvent être comprises entre 0,05 % et 10 %, de préférence entre 0,1 % et 7 % et encore plus préférentiellement entre 0,2 % et 5%, par rapport au poids total de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de l'ammoniaque de la soude ou une (poly)amine primaire, secondaire ou tertiaire comme la monoéthanolamine, la diéthanolamine, la triéthanolamine, l'isopropanolamine ou la propanediamine-1.3, ou encore par ajout d'un acide, de préférence un acide carboxylique tel que par exemple l'acide citrique.

Les compositions détergentes selon l'invention peuvent bien entendu contenir en outre tous les adjuvants usuels comme par exemple des parfums, des agents conservateurs, des séquestrants, des épaississants, des adoucissants, des modificateurs de mousse, des colorants, des agents nacrants, des agents hydratants, des agents antipelliculaires ou anti-séborrhéiques, des vitamines, des filtres solaires siliconés ou non, des agents de mise en suspension, des protéines, des silicones, des céramides, des pseudocéramides, des acides gras à chaînes linéaires ou ramifiées en C₁₆-C₄₀, des hydroxyacides, des électrolytes, des polymères et autres.

Bien entendu, l'homme de l'art veillera à choisir ce ou ces éventuels composés complémentaires et/ou leurs quantités de manière telle que les propriétés avantageuses attachées intrinsèquement à l'association (base lavante + polymère cationique + une silicone spécifique) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques telles que les cheveux consistant à appliquer sur lesdites fibres mouillées une quantité efficace d'une composition telle que définie ci-dessus, puis à effectuer un rinçage à l'eau après un éventuel temps de pause.

Comme indiqué précédemment, les compositions conformes à l'invention confèrent aux cheveux, après rinçage, un remarquable effet coiffant qui se manifeste notamment par une facilité de coiffage et de maintien, ainsi qu'un apport de volume et de légèreté, nettement améliorés.

Un exemple concret, mais nullement limitatif, illustrant l'invention va maintenant être donné.

### EXEMPLE

On a réalisé deux compositions de shampooing, l'une conforme à l'invention (composition A) et l'autre comparative (composition B) :

| | A Invention | B Comparatif |
|---|---|---|
| - Lauryléthersulfate de sodium (C12/C14 à 70/30) à 2,2 moles d'oxyde d'éthylène en solution aqueuse à 28% de MA (MA = matière active) | 14 gMA | 14 gMA |
| - MIRANOL C2M CONC (*) | 3,6 gMA | 3,6 gMA |
| - Polymère cationique (**) | 0,2 g | 0,2 g |
| - Silicone aminée selon l'invention (***) | 2,45 gMA | - |
| - Silicone aminée (****) | - | 2,45 g |
| - Mélange de 1-hexadécyloxy octadodécanol et d'alcool cétylique | 2,5 g | 2,5 g |
| - Monoisopropanolamide d'acides de coprah | 0,8 g | 0,8 g |
| - Acide citrique qs pH | 5 | 5 |
| - Eau déminéralisée qs | 100 g | 100 g |

| | | |
|---|---|---|
| (*) : Cocoamidoéthyl (N-hydroxyéthyl, N-carboxyméthyl) glycinate de sodium, vendu par RHONE POULENC. | | |
| (**) : Gomme de guar modifiée par du chlorure de 2,3-époxypropyl triméthylammonium vendue sous la dénomination JAGUARS C13 S par la société RHONE POULENC. | | |
| (***) : Amodiméthicone de masse moléculaire moyenne en poids d'environ 15 000 vendue en émulsion cationique à 35% de matière active sous la dénomination Silsoft® TP515 Silicone Emulsion " par la Société OSI. | | |
| (****) : Amodiméthicone de masse moléculaire moyenne en poids d'environ 50 000 vendue en émulsion cationique à 35% de matière active sous la dénomination FLUID DC 939 par la société DOW CORNING | | |

On effectue un shampooing en appliquant environ 12 g de la composition A sur des cheveux sensibilisés préalablement mouillés. On fait mousser le shampooing puis on rince abondamment à l'eau.
On procède selon le même mode opératoire que ci-dessus avec la composition comparative B.

Un panel d'experts évalue le démêlage des cheveux séchés, la douceur, le volume, le toucher et le lissage des cheveux séchés.

Tous les experts indiquent une amélioration nette de ces propriétés pour les cheveux traités avec la composition A selon l'invention.

## Revendications

1. Composition détergente et conditionnante, **caractérisée par le fait qu'**elle comprend, dans un milieu cosmétiquement acceptable, (A) une base lavante et (B) un système conditionneur comprenant au moins un polymère cationique et au moins une silicone aminée choisie parmi :
(a) les polysiloxanes dénommés dans le dictionnaire CTFA (4^{ème} Ed. 1991) "amodiméthicone" et répondant à la formule : 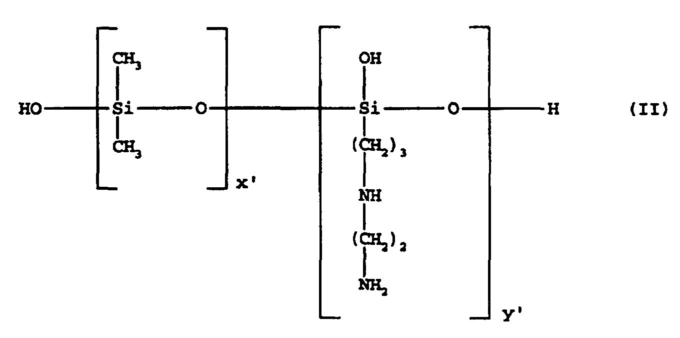 dans laquelle x' et y' sont des nombres entiers dépendant de la masse moléculaire moyenne en poids, telles que ladite masse moléculaire est comprise entre 11 000 et 25 000,
(b) les polysiloxanes dénommés dans le dictionnaire CTFA (7^{ème} Ed. 1997) "amodiméthicone" et répondant à la formule : 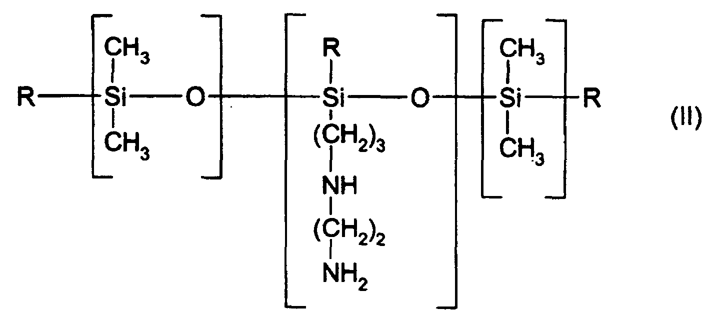 dans laquelle R désigne OH,
la masse moléculaire moyenne en poids étant comprise entre 11 000 et 25 000.

2. Composition selon la revendication 1, **caractérisée par le fait que** ladite base lavante comprend un ou plusieurs tensioactifs choisis parmi des tensioactifs anioniques, amphotères, non ioniques, zwittérioniques, cationiques et leurs mélanges.

3. Composition selon l'une quelconque des revendications 1 ou 2, **caractérisée par le fait que** ladite base lavante est présente à une teneur pondérale comprise entre 4 % et 50 % par rapport au poids total de la composition.

4. Composition selon la revendication 3, **caractérisée par le fait que** ladite teneur est comprise entre 8 % et 35 %.

5. Composition selon la revendication 4, **caractérisée par le fait que** ladite teneur est comprise entre 10 % à 25 %.

6. Composition selon l'une quelconque des revendications précédentes, **caractérisée par le fait que** ledit polymère cationique est présent à une teneur pondérale comprise entre 0,001 % et 10 % par rapport au poids total de la composition.

7. Composition selon l'une quelconque des revendications précédentes, **caractérisée par le fait que** ladite silicone aminée est présente sous forme d'émulsion.

8. Composition selon l'une quelconque des revendications précédentes, **caractérisée par le fait que** ladite silicone aminée est présente à une teneur pondérale comprise entre 0,05 % et 10 % par rapport au poids total de la composition.

9. Composition selon l'une quelconque des revendications précédentes, **caractérisée par le fait que** ledit polymère cationique est choisi parmi les dérivés d'éther de cellulose quatemaires, les cyclopolymères, les polysaccharides cationiques et leurs mélanges.

10. Composition selon la revendication 9, **caractérisée par le fait que** ledit cyclopolymère est choisi parmi les homopolymères du chlorure de diallyldiméthylammonium et les copolymères du chlorure de diallyldiméthylammonium et d'acrylamide.

11. Composition selon la revendication 9, **caractérisée par le fait que** lesdits dérivés d'éther de cellulose quatemaires sont choisis parmi les hydroxyéthylcelluloses ayant réagi avec un époxyde substitué par un groupement triméthylammonium.

12. Composition selon la revendication 9, **caractérisée par le fait que** lesdits polysaccharides cationiques sont choisis parmi les gommes de guar modifiées par un sel de 2,3-époxypropyl triméthylammonium.

13. Composition selon l'une quelconque des revendications précédentes, **caractérisée par le fait qu'**elle présente un pH compris entre 3 et 10.

14. Utilisation d'une composition telle définie à l'une quelconque des revendications précédentes pour le nettoyage et/ou le conditionnement des cheveux.

15. Procédé de lavage et de conditionnement des matières kératiniques telles que les cheveux consistant à appliquer sur lesdites matières mouillées une quantité efficace d'une composition telle que définie à l'une quelconque des revendications 1 à 13, puis à effectuer un rinçage à l'eau après un éventuel temps de pause.

## Patentansprüche

1. Reinigende und konditionierende Zusammensetzung, **dadurch gekennzeichnet, daß** sie in einem kosmetisch akzeptablen Medium (A) eine reinigende Basisformulierung und (B) ein Konditioniersystem enthält, das mindestens ein kationisches Polymer und mindestens ein aminiertes Silicon enthält, das ausgewählt ist unter:
(a) Polysiloxanen, die gemäß CTFA-Nomenklatur (4. Ausgabe 1991) als "Amodimethicon" bezeichnet werden und der folgenden Formel entsprechen: 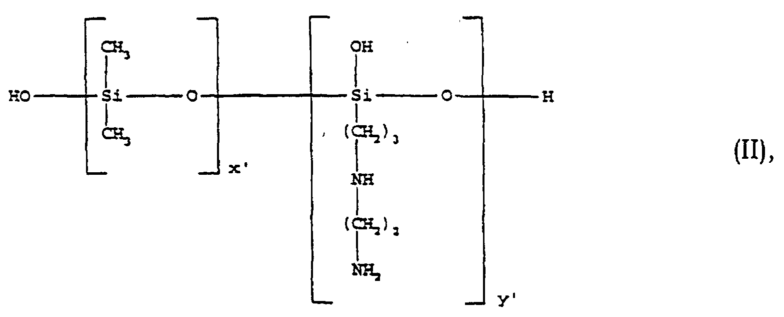 worin x' und y' ganze Zahlen bedeuten, die vom Gewichtsmittel des Molekulargewichts abhängen und so gewählt sind, daß Molekulargewicht im Bereich vón 11 000 bis 25 000 liegt;
(b) Polysiloxanen, die gemäß CTFA-Nomenklatur (7. Ausgabe 1997) als "Amodimethicon" bezeichnet werden und der folgenden Formel entsprechen: 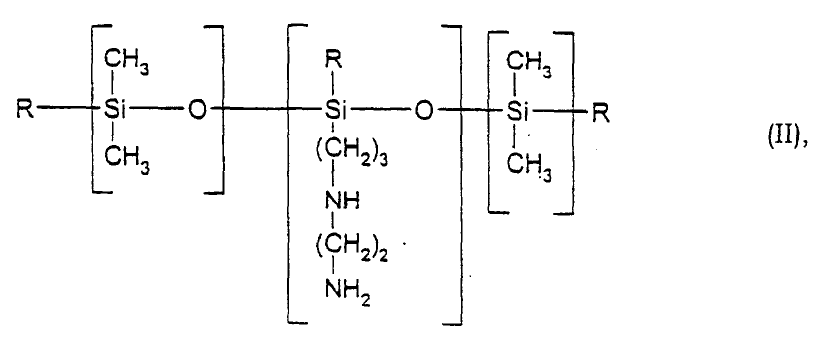 worin R OH bedeutet, wobei das Gewichtsmittel des Molekulargewichts im Bereich von 11 000 bis 25 000 liegt.

2. Zusammensetzung nach Anspruch 1, **dadurch gekennzeichnet, daß** die reinigende Basisformulierung einen oder mehrere grenzflächenaktive Stoffe enthält, die unter den anionischen, amphoteren, nichtionischen, zwitterionischen und kationischen grenzflächenaktiven Stoffen und deren Gemischen ausgewählt sind.

3. Zusammensetzung nach einem der Ansprüche 1 oder 2, **dadurch gekennzeichnet, daß** die reinigende Basisformulierung in einem Gewichtsanteil von 4 bis 50 %, bezogen auf das Gesamtgewicht der Zusammensetzung, vorliegt.

4. Zusammensetzung nach Anspruch 3, **dadurch gekennzeichnet, daß** der Gewichtsanteil im Bereich von 8 bis 35 % liegt.

5. Zusammensetzung nach Anspruch 4, **dadurch gekennzeichnet, daß** der Gewichtsanteil im Bereich von 10 bis 25 % liegt.

6. Zusammensetzungen nach einem der vorhergehenden Ansprüche, **dadurch gekennzeichnet, daß** das kationische Polymer in einem Gewichtsanteil von 0,001 bis 10 %, bezogen auf das Gesamtgewicht der Zusammensetzung, vorliegt.

7. Zusammensetzung nach einem der vorhergehenden Ansprüche, **dadurch gekennzeichnet, daß** das aminierte Silicon in Form einer Emulsion vorliegt.

8. Zusammensetzung nach einem der vorhergehenden Ansprüche, **dadurch gekennzeichnet, daß** das aminierte Silicon in einem Gewichtsanteil von 0,05 bis 10 %, bezogen auf das Gesamtgewicht der Zusammensetzung, vorliegt.

9. Zusammensetzung nach einem der vorhergehenden Ansprüche, **dadurch gekennzeichnet, daß** das kationische Polymer unter den quartären Celluloseetherderivaten, Cyclopolymeren, kationischen Polysacchariden und deren Gemischen ausgewählt ist.

10. Zusammensetzung nach Anspruch 9, **dadurch gekennzeichnet, daß** das Cyclopolymer unter den Homopolymeren von Diallyldimethylammoniumchlorid und den Copolymeren von Diallyldimethylammoniumchlorid und Acrylamid ausgewählt ist.

11. Zusammensetzung nach Anspruch 9, **dadurch gekennzeichnet, daß** die quartären Celluloseetherderivate unter den Hydroxyethylcellulosen ausgewählt sind, die mit einem Epoxid, das mit einer Trimethylammoniumgruppe substituiert ist, umgesetzt wurden.

12. Zusammensetzung nach Anspruch 9, **dadurch gekennzeichnet, daß** die kationischen Polysaccharide unter den Guar-Gummen ausgewählt sind, die mit einem 2,3-Epoxypropyltrimethylammoniumsalz modifiziert sind.

13. Zusammensetzung nach einem der vorhergehenden Ansprüche, **dadurch gekennzeichnet, daß** sie einen pH-Wert im Bereich von 3 bis 10 aufweist.

14. Verwendung einer Zusammensetzung nach einem der vorhergehenden Ansprüche zur Reinigung und/oder zum Konditionieren der Haare.

15. Verfahren zum Waschen und Konditionieren von Keratinsubstanzen, wie den Haaren, das darin besteht, auf die feuchten Keratinsubstanzen eine Zusammensetzung nach einem der Ansprüche 1 bis 13 in einer wirksamen Menge aufzutragen und anschließend gegebenenfalls nach einer Einwirkzeit mit Wasser zu spülen.

## Claims

1. Detergent and conditioning composition, **characterized in that** it comprises, in a cosmetically acceptable medium, (A) a washing base and (B) a conditioning system comprising at least one cationic polymer and at least one aminated silicone chosen from:
(a) polysiloxanes known as "amodimethicone" in the CTFA dictionary (4th Ed., 1991) and corresponding to the formula: 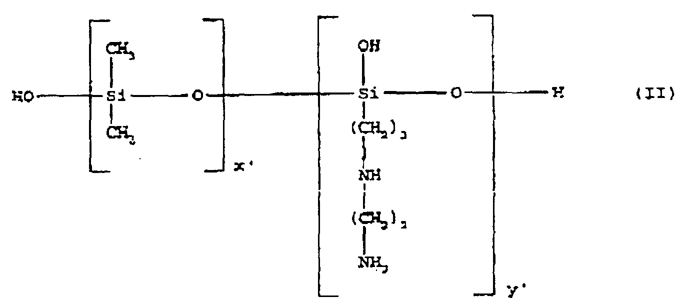 in which x' and y' are integers dependent on the weight-average molecular mass, such that the said molecular mass is between 11 000 and 25 000,
(b) polysiloxanes known as "amodimethicone" in the CTFA dictionary (7th Ed., 1997) and corresponding to the formula: 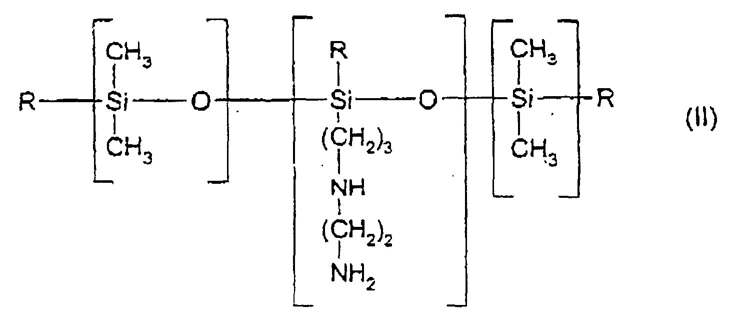 in which R denotes OH,
the weight-average molecular mass being between 11 000 and 25 000.

2. Composition according to Claim 1, **characterized in that** the said washing base comprises one or more surfactants chosen from anionic, amphoteric, nonionic, zwitterionic or cationic surfactants and their mixtures.

3. Composition according to. either one of Claims 1 and 2, **characterized in that** the said washing base is present at a content by weight of between 4% and 50% with respect to the total weight of the composition.

4. Composition according to Claim 3, **characterized in that** the said content is between 8% and 35%.

5. Composition according to Claim 4, **characterized in that** the said content is between 10% and 25%.

6. Composition according to any one of the preceding claims, **characterized in that** the said cationic polymer is present at a content by weight of between 0.001% and 10% with respect to the total weight of the composition.

7. Composition according to any one of the preceding claims, **characterized in that** the said aminated silicone is present in the form of an emulsion.

8. Composition according to any one of the preceding claims, **characterized in that** the said aminated silicone is present at a content by weight of between 0.05% and 10% with respect to the total weight of the composition.

9. Composition according to any one of the preceding claims, **characterized in that** the said cationic polymer is chosen from quaternary cellulose ether derivatives, cyclopolymers, cationic polysaccharides and their blends.

10. Composition according to Claim 9, **characterized in that** the said cyclopolymer is chosen from diallyldimethylammonium chloride homopolymers and copolymers of diallyldimethylammonium chloride and of acrylamide.

11. Composition according to Claim 9, **characterized in that** the said quaternary cellulose ether derivatives are chosen from hydroxyethylcelluloses which have reacted with an epoxide substituted with a trimethylammonium group.

12. Composition according to Claim 9, **characterized in that** the said cationic polysaccharides are chosen from guar gums modified by a 2,3-epoxypropyltrimethylammonium salt.

13. Composition according to any one of the preceding claims, **characterized in that** it exhibits a pH of between 3 and 10.

14. Use of a composition as defined in any one of the preceding claims for cleaning and/or conditioning the hair.

15. Process for washing and conditioning keratinous substances, such as the hair, which consists in applying, to the said wet substances, an effective amount of a composition as defined in any one of Claims 1 to 13 and in then rinsing with water, after an optional leave-in time.
